(19) Europäisches Patentamt
European Patent Office
Office européen des brevets

(11) Publication number: 0 063 021
B1

(12) **EUROPEAN PATENT SPECIFICATION**

(45) Date of publication of patent specification: **31.10.84**

(51) Int. Cl.$^3$: **C 12 Q 1/46,** C 12 Q 1/26

(21) Application number: **82301797.5**

(22) Date of filing: **05.04.82**

(54) **Method for determining the activity of cholinesterases and diagnostioc solution for use therein.**

(30) Priority: **08.04.81 JP 51708/81**

(43) Date of publication of application:
**20.10.82 Bulletin 82/42**

(45) Publication of the grant of the patent:
**31.10.84 Bulletin 84/44**

(84) Designated Contracting States:
**CH DE FR GB IT LI NL**

(56) References cited:
**GB-A-2 018 988**
**US-A-3 959 351**

**CHEMICAL ABSTRACTS, vol. 71, no. 1, 7th July 1969, page 289, col. 1, no. 3153d, Columbus Ohio (USA);**

(73) Proprietor: **FUJIREBIO KABUSHIKI KAISHA also trading as FUJIREBIO INC.**
**6-7, Shimoochiai 4-chome Shinjuku-ku**
**Tokyo 161 (JP)**

(72) Inventor: **Masahara, Yasushi**
**310 Estate Nagayama 3-4-4 Nagayama, 3-chome**
**Tmaoshi Tokyo (JP)**
Inventor: **Ashihara, Yoshihiro**
**28, Harumicho 1-chome**
**Fuchu-shi Tokyo (JP)**
Inventor: **Sugiyama, Masami**
**2-9-29, Motoyokoyamamachi**
**Hachioji-shi Tokyo (JP)**
Inventor: **Harada, Takahiro**
**6-62, Ondacho 1-chome**
**Ube-shi Yamaguchi-ken (JP)**

(74) Representative: **Silverman, Warren et al**
**HASELTINE LAKE & CO. Hazlitt House 28**
**Southampton Buildings Chancery Lane**
**London WC2A 1AT (GB)**

**EP 0 063 021 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method for determining the activity of cholinesterases.

Two kinds of cholinesterase are known these being generally referred to as (1) true cholinesterase, and (2) pseudocholinesterase. True cholinesterase can be found in human red blood cells and human nerve tissue. Pseudocholinesterase can be found in human blood serum and in the human pancreas. Both kinds of cholinesterase decompose (hydrolyse) acylcholines (e.g. acetylcholine) to choline and an acid (acetic acid in the case of acetylcholine decomposition). Insofar as reference is made generally hereinafter to determining the activity of cholinesterase, it is the determination of either cholinesterase which is meant and the term, cholinesterase, as used hereinafter, denotes either true or pseudocholinesterase.

When a person suffers from hepatic disease of anaemia, the amount of cholinesterase in the blood decreases. When a person suffers from nephrosis, diabetes mellitus or a disease of the nervous system, the amount of cholinesterase in the blood increases. A diagnosis of the existance of the diseases mentioned above can be made by measuring the level of cholinesterase in the blood. The accurate determination of the activity of cholinesterase contained in blood serum is therefore significant from both the physiological and clinical viewpoints. The conventional methods for cholinesterase determination include (1) the Takahashi and Shibata method, in which acetyl choline is used as a substrate and the variation of the pH resulting from its decomposition is measured, and (2) a method in which benzoylcholine is used as a substrate, choline liberated from the benzoylcholine is oxidized with choline oxidase to liberate hydrogen peroxide ($H_2O_2$), the hydrogen peroxide is subjected to reaction with phenol and 4-aminoantipyrine in the presence of peroxidase to produce a quinonimine dye, and the concentration of the quinonimine dye produced is colourimetrically measured.

However, such conventional methods have disadvantages. The above method (1) requires complicated steps and the method (2) does not give very precise results.

It is an object of the present invention to provide a simple method for determining the activity of cholinesterase.

According to the present invention, there is provided a method of determining the activity of cholinesterase, comprising the steps of:

mixing a liquid containing a cholinesterase with a substrate solution containing p-methoxybenzoate demethylmonooxygenase, reduced nicotinamide adenine dinucleotide (NADH) and p-methoxybenzoyl chloride cation having the formula:

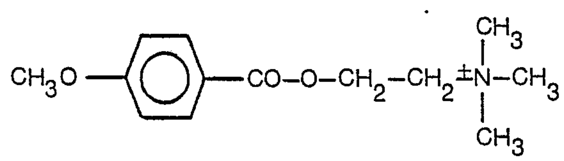 ,

under conditions effective to convert said p-methoxybenzoyl choline to the corresponding p-methoxybenzoic acid and to convert the latter to p-hydroxybenzoic acid while simultaneously converting said NADH to $NAD^+$, and measuring the decrease in absorbance of light caused by NADH, which decrease is caused by conversion of NADH to $NAD^+$, as a measure of the activity of cholinesterase in the liquid.

This invention also provides a diagnostic solution for use in determining the activity of cholinesterase, which comprises, as active ingredients p-methoxybenzoate demethylmonooxygenase, reduced nicotinamide adenine dinucleotide and p-methoxybenzoyl choline cation.

p-Methoxybenzoyl choline which is a substance that provides a cation having the following formula is used as a synthetic substrate:

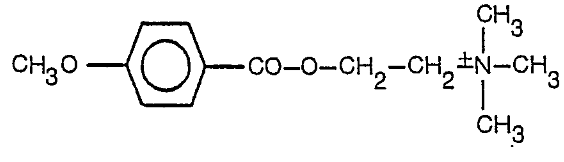 ,

A cholinesterase-containing liquid, such as human blood serum is added to a solution containing a p-methoxybenzoate demethylmonooxygenase, reduced nocotinamide adenine dinucleotide (conventionally and hereinafter referred to as NADH) and the substance providing the aforementioned p-methoxybenzoyl choline cation. p-Methoxybenzoic acid is produced from the p-methoxybenzoyl choline by the action of cholinesterase. The p-methoxybenzoic acid is converted to p-hydroxybenzoic acid by the action of the p-methoxybenzoate demethylmonooxygenase, in the presence of NADH. The consumption of NADH is measured by measuring the decrease in absorbance of light by NADH. The activity of cholinesterase is determined by calculation from the decrease in absorbance of light by NADH.

The following examples illustrate this invention.

Example 1 (Preparative)

The synthetic substrate which is used in the method of the present invention, namely, a substance providing the p-methoxybenzoyl choline cation, was synthesized as follows:

25 g of thionyl chloride were added to 5 g of p-methoxybenzoic acid. After the temperature of the mixture had been kept at 70°C for 6 hours under reflux, the mixture was heated under reduced pressure to remove unreacted thionyl chloride. The residue was dissolved in benzene, and the solution obtained was heated to concentrate it before being cooled to produce a precipitate. The precipitate was recrystallized from benzene to yield p-methoxybenzoyl chloride.

10 Mmol of choline chloride were added to 10 mmol of p-methoxybenzoyl chloride. The temperature of the mixture was kept at 120°C for 5 hours. The residue was washed with ether and 5 g of the residue were dissolved in methanol. The solution was left at a temperature of −20°C for 12 hours and p-methoxybenzoyl choline chloride which cystallized out was separated by filtration.

Example 2

The activity of cholinesterase contained in a human blood serum sample was determined as follows.

5 $\mu$-litre of human blood serum was added to 1 ml of a solution (pH 7.5) containing 0.1 mM of p-methoxybenzoyl choline chloride, 3 U (enzyme activity units) of p-methoxybenzoate demethylmonooxygenase, 0.2 mM of NADH and 100 mM of phosphate buffer. The decrease of the absorbance by the solution was measured at a light wavelength of 340 nm, at a temperature of 30°C, during the course of the reaction. During the determination, use was made of a reaction analyser (LKB 2086 made and sold by Clinicon Co., Sweden). The time lag was 5 seconds and the reaction time was 5 minutes. The enzyme activity unit (U) of cholinesterase was automatically printed out in the analyzer. The calculation activity was performed according to the following formula:

$$U = \frac{\text{Decrease in absorbance at 340 nm}}{\text{Reaction time (5 min)}} \times$$

$$\frac{1}{\text{Molecular extinction coefficient } (\epsilon)} \times \frac{1}{\text{Light-path length (cm)}} \times$$

$$\frac{1.005}{0.005} \times 10^3$$

wherein

$\epsilon = 6.22 \times 10^3$ (Molecular extinction coefficient of NADH)

The results of the measurements, repeated 30 times, were as follows:

Activity unit (U) of cholinesterase = 160 ~ 320 (nmol/ml/min)

Coefficient of variation (CV) = 2.67%.

## Claims

1. A method of determining the activity of cholinesterase, comprising the steps of:

mixing a liquid containing a cholinesterase with a substrate solution containing p-methoxybenzoate demethylmonooxygenase, reduced nicotinamide adenine dinucleotide (NADH) and p-methoxybenzoyl choline cation having the formula:

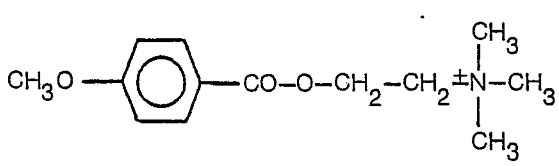

,

under conditions effective to convert said p-methoxybenzoyl choline to the corresponding p-methoxybenzoic acid and to convert the latter to p-hydroxybenzoic acid while simultaneously converting said NADH to $NAD^+$, and measuring the decrease in absorbance of light caused by NADH, which decrease is caused by conversion of NADH to $NAD^+$, as a measure of the activity of cholinesterase in the liquid.

2. A method according to claim 1, wherein the enzyme activity (U) of cholinesterase is calculated in accordance with the formula

$$U = \frac{\text{Decrease in absorbance at 340 nm}}{\text{Reaction time (5 min)}} \times$$

$$\frac{1}{\text{Molecular extinction coefficient } (\epsilon)} \times \frac{1}{\text{Light-path length (cm)}} \times$$

$$\frac{1.005}{0.005} \times 10^3$$

wherein

$\epsilon = 6.22 \times 10^3$ (Molecular extinction coefficient of NADH)

3. A method according to claim 1 or 2, wherein the p-methoxybenzoyl choline cation is provided by p-methoxybenzoyl choline chloride.

4. A diagnostic solution for use in determining the activity of cholinesterase, which comprises, as active ingredients p-methoxybenzoate demethylmonooxygenase, reduced nicotinamide adenine dinucleotide and p-methoxybenzoyl choline cation.

## Revendications

1. Procédé de détermination de l'activité de cholinestérase, qui consiste:

à mélanger un liquide contenant une cholinestérase avec une solution de substrate contenant la p-méthoxybensoate-déméthylmono-oxygénase, le nicotinamide-adénine-dinucléotide réduit (NADH) et le cation de p-méthoxybenzoylcholine répondant à la formule:

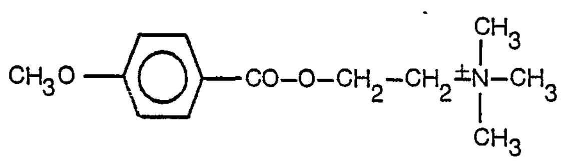 ,

dans des conditions efficaces pour convertir ladite p-méthoxybenzoylcholine en acide p-méthoxy-benzoïque correspondant et pour convertir ce dernier en acide p-hydroxybenzoïque, tout en convertissant simultanément ledit NADH en $NAD^+$, et à mesurer la diminution de la capacité d'absorption de la lumière, provoquée par NADH, diminution qui est due à la conversion de NADH en $NAD^+$, comme mesure de l'activité de cholinestérase dans le liquide.

2. Procédé selon la revendication 1, dans lequel on calcule l'activité enzymatique (U) de cholinestérase selon la formule:

$$U = \frac{\text{diminution du pouvoir absorbant à 340 nm}}{\text{temps de réaction (5 min)}} \times$$

$$\frac{1}{\text{coefficient d'extinction moléculaire } (\epsilon)} \times \frac{1}{\text{longueur du trajet de la lumière (cm)}} \times$$

$$\frac{1{,}005}{0{,}005} \times 10^3$$

dans laquelle

$\epsilon = 6{,}22 \times 10^3$ (coefficient d'extinction moléculaire de NADH).

3. Procédé selon les revendications 1 ou 2, dans lequel le cation de p-méthoxybenzoylcholine est fourni par le chlorure de p-méthoxybenzoylcholine.

4. Solution de diagnostic utilisée dans la détermination de l'activité de cholinestérase, qui comprend, à titre d'ingrédients actifs, la p-méthoxybenzoate-déméthyl-mono-oxygénase, le nicotinamide-adénine-dinucléotide réduit et le cation de p-méthoxybenzoylcholine.

**Patentansprüche**

1. Verfahren zur Bestimmung der Cholinesterase- Aktivität, umfassend die Stufen:

Vermischen einer Cholinesterase enthaltenden Flüssigkeit mit einer Grundlösung, welche Monooxygenase zur Abtrennung der Methylgruppe im p- Methoxybenzoat (p-methoxybenzoate demethylmonooxygenase), reduziertes Nicotinamid- Adenin-Dinucleotid (NADH) sowie das p- Methoxybenzoyl-Cholin-Kation der Formel

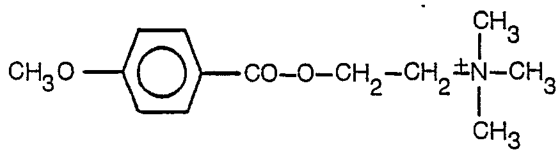
,

enthält unter solchen Bedingungen, daß das p- Methoxybenzoylcholin in die p- Methoxybenzoesäure und letztere in die p- Hydroxybenzoesäure überführt wird und gleichzeitig aus NADH die $NAD^+$- Gruppierung entsteht, sowie Bestimmen der durch die Überführung von NADH in $NAD^+$ verursachten Abnahme von der Lichtabsorption (absorbance of light) des NADH, als Maß für die Aktivität der Cholinesterase in der Flüssigkeit.

2. Verfahren gemäß Patentanspruch 1, wobei die Enzymaktivität (U) der Cholinesterase berechnet wird gemäß der folgenden Formel

$$U = \frac{\text{Abnahme der Absorption bei 340 nm}}{\text{Reaktionszeit (5 min)}} \times$$

$$\frac{1}{\text{mol. Extinktionskoeffizient } (\epsilon)} \times$$

$$\frac{1}{\text{Länge des Lichtwegs (cm)}} \times \frac{1{,}005}{0{,}005} \times 10^3,$$

in welcher $\epsilon = 6{,}22 \times 10^3$ den molekularen Extinktionswert von NADH darstellt.

3. Verfahren nach Patentanspruch 1 oder 2, wobei das p-Methoxybenzoyl- Cholin- Kation vom p-Methoxybenzoyl- Cholin- Chlorid abgeleitet ist.

4. Eine diagnostische Lösung zur Verwendung bei der Bestimmung der Cholinesterase- Aktivität, welche als wirksame Bestandteile Monooxygenase zur Entmethylierung von p- Methoxybenzoat, reduziertes Nikotinamid- Adenin- Dinucleotid und ein p- Methoxybenzoyl- Cholin- Kation enthält.